**Europäisches Patentamt**

(19) **European Patent Office**

**Office européen des brevets**

(11) Publication number: **0 185 404**
**B1**

(12) **EUROPEAN PATENT SPECIFICATION**

(45) Date of publication of patent specification: 02.05.90

(51) Int. Cl.⁵: **C 07 K 15/22,** G 01 N 33/96, G 01 N 33/84, G 01 N 33/72, A 61 K 37/14

(21) Application number: 85201825.8

(22) Date of filing: 08.11.85

(54) **Stroma-free hemoglobin solution, process for the preparation thereof and its application.**

(30) Priority: 09.11.84 NL 8403425

(43) Date of publication of application:
25.06.86 Bulletin 86/26

(45) Publication of the grant of the patent:
02.05.90 Bulletin 90/18

(84) Designated Contracting States:
AT BE CH DE FR GB IT LI NL SE

(56) References cited:
EP-A-0 001 104
EP-A-0 071 888
EP-A-0 078 961
EP-A-0 158 684
US-A-4 358 394

CHEMICAL ABSTRACTS, vol. 95, no. 14, 5th
October 1981, pages 358-359, abstract no.
121024f, Columbus, Ohio, US; L.R. SEHGAL et
al.: "Control of methemoglobin formation in
stroma-free hemoglobin solutions", & J. SURG.
RES. 1981, 31(1), 13-17

(73) Proprietor: EURO-TROL B.V.
Vadaring 74
NL-6702 EA WAGENINGEN (NL)

(72) Inventor: Sprokholt, Ronald
Burg.Hogguerstraat 1143
NL-1064 EJ Amsterdam (NL)
Inventor: Maas, Antonius Henricus Josephus
Zwaardemakerlaan 45
NL-3571 ZB Utrecht (NL)

(74) Representative: Kooy, Leendert Willem et al
OCTROOIBUREAU VRIESENDORP & GAADE
P.O. Box 266
NL-2501 AW The Hague (NL)

(56) References cited:
A. MANUILA et al.: "Dictionnaire français de
médecine et de biologie", vol. 1, page 739,
Masson & Cie., Paris, FR.

CHEMICAL ABSTRACTS, vol. 92, no. 3, 21st
January 1980, page 254, abstract no. 17520s,
Columbus, Ohio, US; K.H. MAYO et al.: "Carp
hemoglobin. V. Effects of pH and P6-inositol on
autooxidation", & BIOCHIM: BIOPHYS. ACTA
1979, 581(1), 44-50

Courier Press, Leamington Spa, England.

## Description

This invention relates to a process for preparing a stroma-free hemoglobin solution, wherein previously purified erythrocytes are lysed by adding distilled or demineralized water, the lysate contents of Ca, Na, K, $HCO_3$ and Cl are adjusted at the values corresponding with those of blood plasma, inositol hexaphosphate is added, the $pCO_2$ and $pO_2$ are adjusted at the desired values by gassing and finally the composition is sterilized by filtering.

Stroma-free hemoglobin solutions and process for the preparation thereof are known, i.a. from EP—A—0 071 888. Such solutions are suitable as blood substitutes during acute blood shortage, but are especially used as standard for calibration and the quality control of determinations of pH, $pCO_2$, $PO_2$ and Hb derivatives in blood.

Determinations of pH, $pCO_2$ and $pO_2$ and hemoglobin derivatives in blood are presently done quite frequently in clinical and other laboratories. The three first-mentioned determinations generally are done by means of specific electrodes and are always relative, that is to say that the unknown sample is compared with a known solution. Similarly the contents of Hemoglobin (Hb) and Hb derivatives are generally determined spectrophotometrically. The quantity of the comparative composition this is of predominating importance.

Said calibration with standard solutions and the quality control thereof should be done every day (and in the United States of North-America it was already suggested to make this legally required) and preferably three solutions are calibrated, one having the relevant parameters at or about the normal value for healthy persons, one said parameters near the lowest value occuring in practice, and one with said parameters near the highest value occuring in practice. An increased or reduced acidity is accompanied by increased or reduced $PCO_2$ and $PO_2$ respectively. Desirable are control-compositions having pH of respectively 7,2, 7,4 and 7,6 having a $PO_2$ of respectively about 130, 100 and 70 mmHg, and a $PCO_2$ of respectively about 60, 40 and 20 mmHg.

Although it is in principle generally knmwn to check a control apparatus by introducing a known composition therein, this causes great problems with blood. Blood is liable to metabolism so that the pH, $pCO_2$, $pO_2$ and the fractions of the Hb-dervatives change. Moreover gas exchange with the surroundings may appear easily, as the partial pressures of carbon dioxide and oxygen deviate strongly from those in the blood. Thus every day a new standard composition should be taken from a closed vessel. After equilibrating or tonometering thereof with a selected gas mixture (mostly these are three different mixtures) the desired values are obtained. For those acquainted with tonometery this will be clear, and the keepability of said standard composition should meet very high requirements. The pH should be within 0.01 of the indicated value, and the $pCO_2$ and $pO_2$ within 1 mmHg of the values given therefor. And this should apply during the guaranteed shelf-life (until the expiration date).

Blood, which is neither conservable nor homogeneous, is unsuitable as calibrating and reference liquid. Hemoglobin solutions, which are obtained by hemolysis of red blood corpuscles (erythrocytes) and centrifuging of the debris and other undissolved material (the stroma) do better. Preparation methods of stroma-free hemoglobin solutions are described in the East-German Patent Specification 150,543, U.S. Patent Specification 4,401,652. and the above-mentioned EP—A—0 071 888.

Although such compositions are homogeneous and consequently already much better they are not stable either. Even if bacterial infection is prevented by asepsis the active homoglobin content decreases rather quickly, mainly in that hemoglobin converts ints (inactive) methemoglobin (an oxidation of FE(II) into FE(III). The affinity of methomoglobin for oxygen is substantially zero.

For improving the oxygen transport of hemoglobin it has been proposed as an optional measure to add 2,3-diphosphoglycerate (2,3 DPG) which is naturally associated with hemoglobin, or pyridoxal phosphate or inositol hexaphosphate $(IHP)_2$, vide page 8, lines 1—4 of the above-cited EP—A—0071888. Applicant has found that addition of inositol hexaphosphate results in a considerable improvement of the stability of the hemoglobin (a dramatic decrease of its conversion into methemoglobin) which seems to have been overlooked until now. The stabilizing effects of 2,3 DPG and pyridoxal phosphate are considerably less.

The above-mentioned EP—A—0071888 provides a process for preparing hemoglobin solutions wherein the erythrocytes are washed at least twice with a solution of a sugar, a sugar-alcohol or a high-molecular substance, are then treated with β-propionolacton at a pH between 7 and 8 and are finally lysed by treating with distilled water. The hemolysate is separated into stroma and hemoglobin solution at a pH between 5.0 and 5.5 and finally the pH of the hemoglobin solution is adjusted at the desired value between 7.2 and 7.6. Optionally, Na, $Cl_2$, $HCO_3$, glucose and/or IHP are added.

This process can be performed in a discontinuously working centrifuge and results in a hemoglobin solution which is guaranteed free from hepatitis virus. However, its physicochemical stability is not complete, as the solution turns turbid when kept for longer times. This turbidity detracts from the value of the hemo-globin solution as it will cast doubt on its sterility, and hence on the correctness of its pH and other concentration values. This turbidity is also detrimental because the hemoglobin solution will then no longer be absolutely safe for use as blood substitute because of the danger of blocking the glomeruli of the kidney. The turbidity is in fact a protein precipitate. Moreover the presence of appreciable amounts of sugar (which may be any sugar other than glucose) or sugar-alcohol may be a nuisance for some applications.

Now it was found that the above objections are overcome, and an absolutely stable hemoglobin solution is obtained which will keep fully clear (i.e. without any trace of haze) for indefinite times (at least for one year if kept under refrigeration) if after lysis inositol hexaphosphate is added and subsequently the pH of the lysate is adjusted between 8.0 and 8.1 with dilute NaOH, a precipitate is removed by centrifuging, the pH is then adjusted between 6.9 and 7.0 with dilute HCl and again precipitate is removed by centrifuging, so much IHP being added that the molar ratio IHP:Hb is from 0.1:1 to 1.0:1.

It appeared that in a thus prepared solution immediately after mixing less than 2% of the total amount of hemoglobin is methemoglobin and, after keeping it at 4°C or lower during one year, less than 7%. The $p_{50}$ (i.e. the oxygen tension with 50% load of the total hemoglobin with oxygen) decreases somewhat in that methemoglobin has an extremely low P50. If 5% of the hemoglobin converts into methemoglobin the P50 decreases with 0.5 mmHg (67 PA). In any case the P50 of the composition when keeping it in the refrigeration will decrease in six months no more than 2 mmHg (267 pA).

A combination of hemoglobin with inositol hexaphosphate was, as far as we are aware of, only mentioned before in an article by P. M. Breepoel et al in Pflügers Archiv *389* (1981) 227—235, wherein it is shown that IHP as well as DGP and ATP (adenosintriphosphate) reduces the oxygen affinity of bovine hemoglobin. However the differences were small, and the stability of similar combinations had not been examined. The molar ratio IHP/HB of the solution described in said article was 2:1.

Furthermore it was found that the conversion of hemoglobin into methemoglobin, already low because of the inositol hexaphosphate, can be retarded further by using the enzyme methemoglobin reductase, which occurs naturally in erythrocytes and consequently in the hemoglobin solution according to the invention, said enzyme reverts the "Irreversible" conversion of hemoglobin→methemoglobin. Required therefore is the presence of NADH (reduced form of nicotine adenine dinucleotide) of which so much has to be present that the molar ratio NADH/Hb is from 0.25:1 to 1.50:1, optimal is a molar ratio NADH/Hb of about 0.5:1 instead of NADH also NADPH (the reduced form of nicotine adenine dinucleotide phosphate) may be present. The addition of NADH to a hemoglobin solution has already been disclosed by Sehgal et al in J. Surg. Res. *31*(1)(1981) 13—17 (vide Chemical Abstracts *95* (1981) 358—359, NR 121025F).

The hemoglobin concentration of the composition will be generally in the range from 3 mMol/l to 15 mMol/l; preferably it is about 8 mMol/l.

The contents of sodium, potassium, chloride and optionally other ions will be within the physiological ranges. The bicarbonate content will be determined by the desired pH and the desired $pCO_2$.

In oxygen-buffering capacity said composition is equal to blood, and the p50 reacts to a shift of the pH in about the same way as of blood, if any.

The p50 is also sensitive to the IHP content, herewith the p50 can be adjusted at normal at those acid-base conditions that are characterizing for acidosis, normal conditions and alkalosis. It is also preferable, as is indicated above, to provide the calibration and reference liquids according to the invention in series of three, one with pH, $pCO_2$ and $pO_2$ at or about the normal values for healthy persons, one with said parameters near the lowest value occurring in practice, and one with said parameters near the highest value occurring in practice.

When performing the process of the invention the erythrocytes are first purified by repeated rinsing with an isotonic or weakly hypertonic NACl solution and preferably they are also previously stripped of leucocytes by feeding a suspension thereof through a column with a suitable absorbent (1.g. "Erypur" of the organon company). This was known i.a. from the above-mentioned U.S. Patent Specification 4,401,652 the lysis is realised by bringing the purified erythrocytes into a minimum of two volumes but preferably in four volumes of a strongly hypotonic solution, preferably sterile distilled or demineralized water, which, as precaution, generally also contains some bactericide. If desired the lysis can be speeded up with ultrasonic vibrations but this is not essential, preferably the lysate is centrifuged, so that the stroma (debris of the erythrocyte cell walls and other solids) is removed. The supernatant is evacuated carefully from the sediment by means of a siphon. If desired said lysate is concentrated, which e.g. can be done by evaporation but preferably by ultrafiltration, e.g. by means of shafts of hollow fibres. The hemoglobin yield may be as high as 90—95%.

To said solution the ions $NA^+$, $K^+$, $CA^{2+}$, $MG^{2+}$, $HCO^-4^3$ and $Cl^-$ are added as salts, in such amounts that the final solution will have the desired concentrations of sodium, potassium, magnesium and chloride. By previous careful calculation the amount of bicarbonate the pH will then be at the desired value, and also inositol hexaphosphate is added in such an amount that the molar ratio IHP/Hb is from 0.1:1 to 10:1. Optionally together with the IHP also NADH is added, preferably in such an amount that the molar ratio NADH/Hb becomes about 0.5:1. Often it will be necessary to centrifuge the solution once again to obtain a really clear final composition. Finally by gassing with a mixture of nitrogen, carbon dioxide and oxygen the $pCO_2$ and $pO_2$ are adjusted at the correct values.

The invention also relates to a stroma-free hemoglobin solution obtained by the above-indicated process.

Finally the invention also relates to the application of said stable composition. In the first place it may be used when determining the pH, $pCO_2$, $pO_2$ and/or hemoglobin content in blood, this determination now being calibrated with a solution as described above. As is already said this has the advantages that also after keeping it for a long time an absolutely reliable composition is obtained which can be used without any precaution and which is also free of haze.

But such a hemoglobin solution may also be used as blood substitute having the advantage that it does not show immunogenity (by the absence of the erythrocyte cell walls). On the other hand it will be excreted very quickly in that the kidneys do not retain at all hemoglobin not locked within the red blood corpuscles. Thus it is specially considered for infusion in emergency cases.

Further it may also be used as standard for the (spectrophotometrical) determination of hemoglobin and Hb derivatives.

The invention is now elucidated by the following examples.

Example I

All actions were done under sterile conditions (laminair airflow cabinet, class 100; sterile solutions, etc.).

1) Human blood was collected in ACD solution, portions of 420 ml in bottles of 500 ml in which already 80 ml of ACD solution was present, totally 6 liters.

2) Said bottles were placed in centrifuge cups and centrifuged at 3000 ×G during 30 minutes. The supernatants were evacuated through siphons per bottle 200—250 ml of cells were recovered.

3) The blood cells were suspended in 0.9% NACI solution, final volume 500 ml per bottle.

4) The blood cell suspensions were fed through a column "Leucofilter" (erypur of the Organon Company) so that the white blood cells and a greater part of the blood platelets were removed.

5) The blood cell suspensions were centrifuged at 3000 ×G during 30 minutes. The supernatants were removed with a siphon.

6) Similarly to (3) making up to 500 ml per bottle was done with 0.9% NaCl solution.

7) Similarly to (5) centrifuging was done again at 3000 G during 30 minutes, the supernatants were discarded.

8) Per bottle there were now about 200 ml of erythrocytes.

9) All portions of erythrocytes were combined and mixed well, and subsequently distributed over bottles of 3 liters, each bottle containing 0.6 liter of erythrocytes. Now to each bottle 2.4 liters of sterile, distilled water was added and mixed. Also per liter 1 G of neomycin sulphate (dissolved in about 2 ml water) was added (to prevent bacterial growth). Said mixtures were kept in a refrigerator at +4°C during one night.

10) The cooled hemoglobin solution was distributed at room temperature over polycarbonate centrifuge bottles (sterile, with a special closure). Herein they were centrifuged at 0—10°C at 27,000 G in a sorvall RC-5 centrifuge with GSA-rotor during 30 minutes.

11) The above liquids were sterilely evacuated and collected in bottles of 3 liters.

12) Hereto $CACl_2$ and NACI were added at concentrations of resp. 0.02 M and 0.13 M and subsequently 8 ml of 0.25 m sodium inositol hexaphosphate solution were added to 3 liters, the pH of which being adjusted at 8,0 with dilute HCl, so that IHP/Hb was now 0.5:1. Now 1 m NAOH was added until the pH was 9.05. A precipitate formed (CA—IHP, membrane proteins).

The IHP-NA-salt had been purchased from Sigma Chemicals (St. Louis, MO, 63178, USA).

13) In a sorvall centrifuge centrifuging was done at 27,000 G during 20 minutes.

14) The above hemoglobin solutions were carefully evacuated and combined.

15) With 1 M HCl the pH was adjusted at 7,0. Again a precipitate formed (protein).

16) Again centrifuging was done at 27,000 G during 30 minutes.

17) The above hemoglobin solutions were evacuated and combined.

18) Previous to concentrating the solution was now filtered through pre-filters and ultra-filters having pores of 0.8 μm.

19) The solution was concentrated by ultrafiltration by means of shafts of hollow fibres (apparatus Amicon DC-2 with H1P10-8 cartridges). This was continued until a hemoglobin concentration of $10^{-2}$ M was attained.

20) Now NADH in water was added, about 7 G in total, so that the molar ratio NADH/Hb was now 0,5:1.

21) Now Hb, $pH$,, $pCO_2$, NA, K, Cl and free-ionised calcium determined directly measured and the pH, $pCO_2$ and the Bi carbonate content were determined with the Astrup technique.

22) The obtained liquid was divided into three roughly equal parts. From the results of the determinations (21) it was calculated how much of the various ions should be added to obtain a composition as near as possible to that of blood plasma.

23) now NACI, KCl, $CACl_2$ and $NAHCO_3$ were added, so that in each portion the relation between pH and $pCO_2$ was known.

(pBAR=760 MMHg)
=101,3 kPA

| pH | $pCO_2$ |
|------|-------------------|
| 7.20 | 60 MMHG (8.00 kPA) |
| 7.40 | 40 MMHG (5.33 kPA) |
| 7.60 | 20 MMHG (2.67 kPA) |

24) To each portion so much IHP was added that for each $pH/pCO_2$ combination the P50 was about 26.5 MMHG (3.53 kPA) (for this purpose add three different amounts of IHP and determine the $PO_2$ at a constant $pCO_2$, whereafter by intrapolation the desired IHP concentration is found; P50 is a function of IHP/Hb).

25) Then centrifuging was done again in a Sorvall centrifuge at 27,000 G during 20 minutes.

26) The above hemoglobin solution was evacuated and sterilized by filtration through an ultrafilter having pores of 0.22 μM.

27) The product was sterilely distributed over bottles with rubber lugs.

The product contains $8,0 \cdot 10^{-3}$ M of Hb, of which less than 2% was methemoglobin (after 1 year in the refrigerator less than 7%). The hemoglobin yield in this case was about 60%.

It contained 0.21 M of NA; 0.011 M of K˙ and 0.16 M of CL'. It contained extremly few discrete particles, which anyhow were smaller than 0.22 μm. After 251-fold diluted with HICN reagent it was found;

$$E^{1\,cm}_{750\,nm} \qquad <0.0002 \text{ and } E_{540}/E_{514}=1.59.$$

The relation between P50 and pH was the same as is known from the literature, and the temperature coefficients for pH, $pCO_2$ and $pO_2$ were approximately those of blood.

Example II

1) Bovine blood was made non-clotting with a heparin solution (550 ml of blood+3 ml of solution).

2)—11) Exactly as with Example I.

12) To the hemoglobin solution $CACl_2$ and NACI were added at concentrations of resp. 0.02 M and 0.24 M and subsequently in total about 3.5 ml of 0.25 m NA-IHP the pH of which was first adjusted at 8.0 with dilute HCl. The pH of this mixture was 7.4.

13) A slight haze was removed by centrifuging at 27,000 G during 20 minutes.

14)—24) were exactly as (17)—(27) of example I.

The properties of this composition were substantially equal to those of the composition of Example I. Only after keeping in the refrigator for half a year it had a very slight haze.

Example III

1) Bovine blood was made non-clotting with a heparin solution (550 ml of blood +3 ml of solution).

2)—27) In accordance with Example I.

The properties of said composition were substantially equal to those of the composition of example 1, also that even after half a year it did not show any trace of haze.

## Claims

1. Process for preparing a stroma-free hemoglobin solution, wherein previously purified erythrocytes are lysed by adding distilled or demineralized water, the lysate contents of Ca, Na, K, $HCO_3$ and Cl are adjusted at the values corresponding with those of blood plasma, inositol hexaphosphate is added, the $pCO_2$ and $pO_2$ are adjusted at the desired values by gassing and finally the composition is sterilized by filtering, characterized in that after inositol hexaphosphate has been added the pH of the lysate is adjusted between 8.0 and 8.1 with dilute NaOH, a precipitate is removed by centrifuging, the pH is then adjusted between 6.9 and 7.0 with dilute HCl and again a precipitate is removed by centrifuging, so much IHP being added that the molar ratio IHP/Hb is from 0.1 to 1.0:1.

2. Process according to Claim 1, characterized in that methemoglobin reductase and reduced NAD (NADH) are added to the composition.

3. Process according to Claim 1 or 2, characterized in that by diluting and/or concentrating the eventual hemoglobin concentration is adjusted at 3 to 15 mmol/litre.

4. Process according to any of the preceding Claims, characterized in that the molar ratio IHP/Hb is adjusted at 0.5:1.

5. Process according to any of the preceding Claims, characterized in that three comparable solutions are prepared, in one of them the pH being adjusted at about 7.2, in another at about 7.4 and in the third one at about 7.6.

6. Stroma-free hemoglobin solution, obtained by a process of any of the Claims 1 through 5.

7. Process for determining pH, $pCO_2$, $pO_2$ and/or Hb in blood, characterized in that said determination is calibrated with a stroma-free hemoglobin solution obtained by a process of any of the Claims 1 through 5.

8. Stroma-free hemoglobin solution, obtained by a process of any of the Claims 1 through 5, for use as a blood substitute.

## Patentansprüche

1. Verfahren zur Herstellung einer stromafreien Hämoglobinlösung, wobei vorher gereinigte Erythrocyten durch Zugabe von destilliertem oder entmineralisiertem Wasser lysiert werden, der Gehalt des Lysats an Ca, Na, K, $HCO_3$ und Cl auf die Werte gebracht wird, die denen von Blutplasma entsprechen, Inositolhexaphosphat zugegeben wird, die $pCO_2$- und $pO_2$-Werte durch Begasung auf die gewünschten Werte gebracht werden und schließlich die Zusammensetzung durch Filtrieren sterilisiert wird, dadurch gekennzeichnet, daß nach Zugabe von Inositolhexaphosphat der pH-Wert des Lysats mit verdünnter NaOH auf einen Wert zwischen 8,0 und 8,1 eingestellt wird, ein Niederschlag durch Zentrifugieren entfernt wird, der pH-Wert dann mit verdünnter HCl auf einen Wert zwischen 6,9 und 7,0 eingestellt und wiederum ein Niederschlag durch Zentrifugieren entfernt wird, und soviel IHP hinzugefügt wird, daß das Molverhältnis IHP/Hb 0,1 bis 1,0:1 beträgt.

2. Verfahren nach Anspruch 1, dadurch gekennzeichnet, daß Methämoglobin-Reduktase und reduziertes NAD (NADH) der Zusammensetzung zugegeben werden.

3. Verfahren nach Anspruch 1 oder 2, dadurch gekennzeichnet, daß die jeweilige Hämoglobinkonzentration durch Verdünnen und/oder Konzentrieren auf 3 bis 15 mMol/Liter eingestellt wird.

4. Verfahren nach einem der vorangehenden Ansprüche, dadurch gekennzeichnet, daß das Molverhältnis IHP/Hb auf 0,5:1 eingestellt wird.

5. Verfahren nach einem der vorangehenden Ansprüche, dadurch gekennzeichnet, daß drei vergleichbare Lösungen hergestellt werden, wobei in einer der Lösungen der pH-Wert auf etwa 7,2, in einer anderen Lösung auf etwa 7,4 und in der dritten Lösung auf etwa 7,6 eingestellt wird.

6. Stromafreie Hämaglobinlösung, erhalten nach einem Verfahren nach einem der Ansprüche 1 bis 5.

7. Verfahren zur Bestimmung des pH-, pCO₂-, pO₂- und/oder Hb-Wertes im Blut, dadurch gekennzeichnet, daß diese Bestimmung mit einer stromafreien Hämoglobinlösung geeicht wird, die durch ein Verfahren gemäß einem der Ansprüche 1 bis 5 erhalten wurde.

8. Stromafreie Hämoglobinlösung, erhalten durch ein Verfahren nach einem der Ansprüche 1 bis 5 zur Verwendung als Blutersatz.

**Revendications**

1. Procédé pour préparer une solution d'hémoglobine exempte de stroma, dans lequel des érythrocytes préalablement purifiés sont lysés par addition d'eau distillée ou déminéralisée, les teneurs du lysat en Ca, Na, K, HCO₃ et Cl sont ajustées aux valeurs correspondant à celles du plasma sanguin, de l'hexaphosphate d'inositol est ajouté, pCO₂ et pO₂ sont ajustés aux valeurs désirées par absorption de gaz, et enfin la composition est stérilisée par filtration, caractérisé en ce que, après l'addition d'hexaphosphate d'inositol, le pH du lysat est ajusté entre 8,0 et 8,1 avec NaOH diluée, un précipité est éliminé par centrifugation, le pH est alors ajusté entre 6,9 et 7,0 avec HCl dilué et à nouveau un précipité est éliminé par centrifugation, la quantité d'HPI ajoutée étant telle que le rapport molaire HPI/Hb vaut de 0,1/1 à 1,0/1.

2. Procédé selon la revendication 1, caractérisé en ce que l'on ajoute à la composition de la méthémoglobine réductase et de la forme réduite de NAD (NADH).

3. Procédé selon la revendication 1 ou 2, caractérisé en ce que la concentration finale d'hémoglobine est ajustée, par dilution et/ou concentration, entre 3 et 15 millimoles/litre.

4. Procédé selon l'une quelconque des revendications précédentes, caractérisé en ce que le rapport molaire HPI/Hb est ajusté à 0,5/l.

5. Procédé selon l'une quelconque des revendications précédentes, caractérisé en ce que l'on prépare trois solutions comparables, le pH étant ajusté à 7,2 environ dans l'une d'elles, à 7,4 environ dans une autre et à 7,6 environ dans la troisième.

6. Solution d'hémoglobine exempte de stroma, obtenue par un procédé selon l'une quelconque des revendications 1 à 5.

7. Procédé pour déterminer le pH, le pCO₂, le pO₂ ou la quantité de Hb dans le sang, caractérisé en ce que l'étalonnage pour ladite détermination est effectué à l'aide d'une solution d'hémoglobine exempte de stroma, obtenue par un procédé selon l'une quelconque des revendications 1 à 5.

8. Solution d'hémoglobine exempte de stroma, obtenue par un procédé selon l'une quelconque des revendications 1 à 5, à utiliser comme substitut du sang.